# EUROPEAN PATENT SPECIFICATION

(11) **EP 2 910 195 B1**
(45) Date of publication and mention of the grant of the patent: **30.12.2020**
(21) Application number: 15162654.6
(22) Date of filing: 24.09.2012
(51) Int. Cl.: A61B 17/04, A61B 17/88

(54) **COMPLIANT INSERTER FOR IMPLANTS**
BIEGSAMER IMPLANTATEINFÜHRER
DISPOSITIF D'INSERTION SOUPLE POUR IMPLANTS

(30) Priority: 23.09.2011 US 201113242085
(43) Date of publication of application: 26.08.2015
(62) Document number(s) of the earlier application(s) in accordance with Art. 76 EPC: 12185683.5 / 2 572 651
(73) Proprietor: DePuy Synthes Products, Inc., Raynham, MA 02767 (US)
(72) Inventor: Sengun, Mehmet Ziya, Raynham, MA Massachusetts 02767 (US); Piccirillo, Justin M., Raynham, MA Massachusetts 02767 (US); Miller, Gerome, Raynham, MA Massachusetts 02767 (US); Stephen, Arthur G., Raynham, MA Massachusetts 02767 (US)
(74) Representative: Carpmaels & Ransford LLP

(56) References cited:
- US-A- 5 464 407

## Description

### BACKGROUND OF THE INVENTION

### 1. Field of the Invention

The invention relates to tools for inserting implants into bone and more particularly to drivers for suture anchors.

### 2. Description of the Related Art

A common injury, especially among athletes, is the complete or partial detachment of tendons, ligaments or other soft tissues from bone. Tissue detachment may occur during a fall, by overexertion, or for a variety of other reasons. Surgical intervention is often needed, particularly when tissue is completely detached from its associated bone. Currently available devices for tissue attachment include screws, staples, suture anchors and tacks.

Arthroscopic tissue attachment is commonly practiced in shoulder rotator cuff and instability procedures. Typically, an anchor loaded with suture is fixated to bone using an inserter-type device. The suture is normally slidably attached to the anchor through an eyelet or around a post, such that a single length of suture has two free limbs. The suture limbs typically are carried along the exterior of the inserter, usually within a groove or other exterior channel, or within the interior of the inserter. After the anchor is inserted into the bone, one limb of the suture is passed through soft tissue to be repaired such as a tendon or labrum. The two ends of the suture are then tied to each other, thereby capturing the soft tissue in a loop with the anchor. Upon tightening the loop, the soft tissue is approximated to the bone via the anchor.

Some anchor inserters are utilized to push an anchor into a pre-drilled hole in a bone, such as shown in U.S. Patent No. 7,381,213 by Lizardi, while other inserters apply torque to a helically threaded anchor to rotate the anchor into a pre-formed hole, such as illustrated in U.S. Patent Publication No. 2008/0147063 by Cauldwell et al. Certain anchors are asymmetrical and require relatively small force for insertion, while other, symmetrical anchors must be driven at a higher forces to ensure sufficient fixation between the anchor and the bone. Some anchors benefit from internal support by a substantially rigid tip of an inserter while being driven into bone.

The tip of an implant driver typically is constructed of a hardened material which may be strong but brittle. In other words, the driver tip usually lacks ductility. Large strains imparted on a material may result in failure of the implant and/or the driver tip. Failure of the driver tip may result in breakage which must be retrieved from the patient. Strains may arise when an implant is being inserted at an off-axis angle into a hole in bone, or when the implant is partially or fully inserted and a laterally-directed load is applied to the driver handle.

One type of asymmetric, push-in anchor is disclosed by Pedlick et al. in U.S. Patent No. 6,961,538. In one embodiment, an installation tool has a substantially rigid elongated rod with a somewhat flexible distal end that encourages a plow-like edge of a wedge-shaped anchor to dig into the wall of a pre-formed hole in bone. The installation tool does not appear to be capable of providing any internal support to the anchor during insertion.

Another type of asymmetric anchor is described by Lizardi in U.S. Patent No. 6,527,795. A tapered anchor has a flared portion on its trailing end. An insertion tool may include a flexible portion that allows the tool to bend when pressure is exerted to generate a compressive force that is greater on one side of the anchor, which toggles or rotates the anchor.

A further consideration is having sufficient yet releasable attachment between the anchor and the inserter. Some systems rely on an interference fit, while others utilize a threaded engagement. A implant inserter device is described by Stoffel et al. in U.S. Patent Publication No. 2010/0292704 having an inner sleeve which collapses from an undeformed position to a deformed position to grip an implant.
US 5464407 forms the basis of the two-part form of claim 1 and discloses a surgical screwdriver comprising a shaft, a portion of which is flexible as it is formed from centric coil springs.

It is therefore desirable to have an improved inserter which can apply sufficient insertion force to an implant without damaging the implant and without breakage.

### SUMMARY OF THE INVENTION

The present invention provides an inserter according to claim 1 and a combination according to claim 9. An object of the present invention is to provide an improved inserter which concentrates bending forces in a localized region to control deformation and minimize breakage.

Another object of the present invention is to provide such an inserter which can generate internal support for an implant.

This invention features an implant inserter including an elongated, substantially rigid shaft having a proximal surface which is capable of receiving a driving force, and a substantially rigid tip portion having a distal end and a proximal end. At least the distal end of the tip portion is capable of being placed within an implant. The inserter further includes a compliant region disposed between the tip portion and the shaft which requires less lateral force to bend than the tip portion.

In some embodiments, the tip portion is capable of providing internal support for the implant during insertion into bone, and the compliant region is formed of a material that is more malleable than that of the tip portion In certain embodiments, the distal tip is capable of applying torque to the implant.
In a number of embodiments, the inserter is placed within a package and sterilized, the sterilization occurring before, during or after being placed in the package. The implant such as a suture anchor is placed within the same package in some embodiments and in a different sterilizable package in other embodiments. The package maintains sterility of the inserter and of the implant if present.

In some embodiments, the inserter further includes at least one implant retention element carried by the tip, such as a laterally-extending projection formed on the tip. In one embodiment, the projection has a smooth distalleading edge and a sharply-defined transition at a trailing edge.

A method of inserting a suture anchor into bone, which is herein disclosed and which does not form part of the invention, may include selecting a suture anchor having a distal end and a proximal end, and defining a lumen open at the proximal end. The method further includes coupling the suture anchor to an inserter including an elongated, substantially rigid shaft having a proximal surface which is capable of receiving a driving force, a substantially rigid tip portion having a distal end and a proximal end, and at least the distal end capable of being placed within the lumen of the suture anchor to provide internal support for the suture anchor during insertion into bone, the inserter further including a distal- facing shoulder at the proximal end of the tip portion capable of engaging the proximal end of the suture anchor, and a compliant region disposed between the shoulder and the shaft which requires less lateral force to bend than the tip portion, at least when the tip portion has been placed within the suture anchor. The suture anchor is then driven into the bone.

### BRIEF DESCRIPTION OF THE DRAWINGS

In what follows, preferred embodiments of the invention are explained in more detail with reference to the drawings, in which:
FIG. 1 is a schematic side view of an inserter according to the present invention releasably attached to a suture anchor which has been driven into bone;
FIG. 2 is an enlarged view of a portion of the inserter of FIG. 1 featuring a bellows-type compliant region;
FIG. 3 is a side view of an exemplary inserter not forming part of the present invention having a reduced-diameter neck region;
FIG. 4 shows the inserter of FIG. 3 internally supporting a suture anchor;
FIG. 5 is a schematic perspective view of yet another exemplary inserter not forming part of the present invention having an anchor retention element;
FIG. 6 is an enlarged cross-sectional view of the inserter of FIG. 5;
FIG. 6A is an enlarged view of a portion of FIG. 6; and
FIG. 7 is a schematic cross-sectional view of an anchor retention element interacting with an internal wall of an implant.

### DETAILED DESCRIPTION OF THE PRESENTLY PREFERRED EMBODIMENTS

This invention may be accomplished by an implant inserter with an elongated, substantially rigid shaft having a proximal surface which is capable of receiving a driving force, and a substantially rigid tip portion having a distal end and a proximal end. The tip portion is capable of being placed within an implant. The inserter further includes a compliant region disposed between the tip portion and the shaft which requires less lateral force to bend than the tip portion, at least when the tip portion has been placed within the implant. The term "bend" includes elastic, plastic and irreversible deformations of the compliant region, but does not include fracture or other breakage thereof.

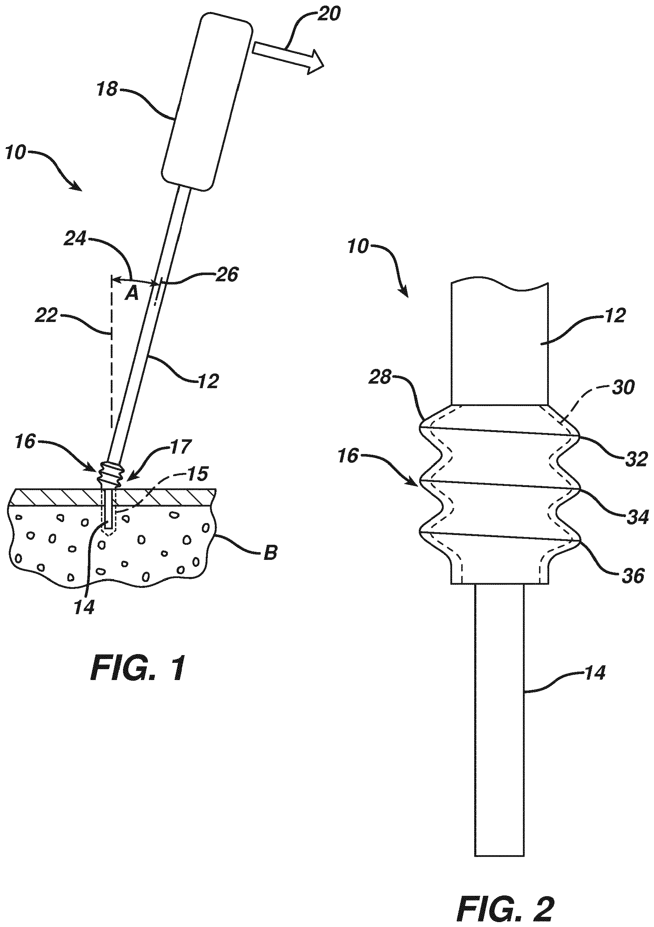

FIG. 1 illustrates a compliant inserter 10 according to the present invention having an elongated rigid shaft 12, a substantially rigid tip portion 14, and a compliant region 16. Shaft 12 has a proximal surface including a handle 18 to which an axial and/or rotational driving force is applied. Tip portion 14 is shown substantially within a lumen of a suture anchor 15, illustrated in phantom, that has been driven by inserter 10 into a hole or bore 17 in bone B of a patient.

Shaft 12, with centerline 26, is shown off-axis by an angle A relative to tip portion 14 and anchor 15 in hole 17, represented by bore axis 22. This orientation may be caused by an approach during insertion that is not perpendicular to the axis of the hole 17 in bone B, or by lateral force, represented by arrow 20, applied to handle 18. It is desirable to accommodate misalignment during insertion of up to thirty degrees off-axis.

Compliant region 16 is shown in greater detail in FIG. 2 in an unstressed, axially-aligned condition relative to shaft 12 and tip portion 14. Compliant region 16 serves as a flexure between shaft 12 and tip portion 14 to provide strain relief between these components, that is, it provides greater structural compliance to bending than the other components, especially the tip portion 14, at least when tip portion 14 has been placed within an implant to be inserted. In this construction, compliant region 16 is a hollow bellows 28 which includes a series of convolutions 32, 34 and 36. Inner bellows wall 30 is shown in phantom.

In some constructions, bellows 28 is a cylindrical symmetrical metal bellows such as a sylphon formed by metal spinning onto a mandrel or by hydrostatic forming within a mold. In other constructions, the compliant region is an asymmetric bellows or other structure, and includes corrugations and other variations in cross-sectional shapes to provide a region that bends or otherwise yields more readily than does the tip portion 14 of the inserter 10, at least when tip portion 14 has been placed within an implant to be inserted. Corrugations, channels or other structures may be helical, parallel or with variable skew in orientation. Corrugations and other structures may be circular, oval or polygonal cross-section, or any combination thereof. In one construction, the compliant region includes a helical spring-type element.

In certain constructions, compliant region 16 transmits torque in at least one rotational direction to enable a helically threaded anchor to be driven into bone. Anchor 15 has a hexagonal cross-sectional shape in one construction, such as disclosed by Cauldwell et al. in U.S. Patent Publication No. 2008/0147063. Suitable implant materials include those disclosed by Cauldwell et al. and in U.S. Patent No. 7,381,213 by Lizardi.

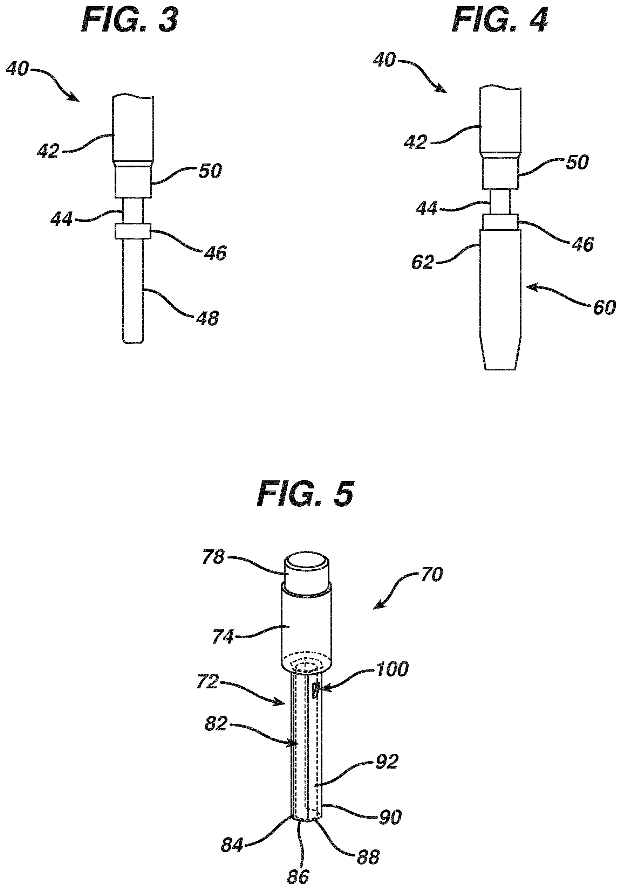

An exemplary inserter 40 is shown in FIGS. 3 and 4 having a shaft 42, a compliant region 44 formed by a reduced-diameter neck, a shoulder 46, and a tip portion 48. The shoulder 46 is shown in FIG. 4 abutting the proximal end 62 of anchor 60, with tip portion 48 fully occupying a lumen within anchor 60. The proximal end of shaft 42 is not illustrated.

In this construction, all components are hollow metal cylinders with a central lumen extending therethrough, and shaft 42 has a transition region 50 which is approximately the diameter of shoulder 46. Tip portion 48 has a wall thickness of 0.15 mm (0.006 inches) and neck 44 has a length of 2 millimeters and a wall thickness the same or slightly greater than that of tip portion 48, preferably about 0.25 mm (0.010 inches). A currently preferred material for neck 44 and tip portion 48 is stainless steel 304 annealed. A suitable material for shaft 42 is stainless steel 17-4 hardened to H900.

Although neck 44 may appear to be less bendable than tip portion 48 when inserter 40 is viewed alone as shown in FIG. 3, the relative lengths, wall thicknesses and materials of inserter 40 are selected relative to dimensions and material of a chosen implant to ensure that bending will first occur along the length of neck 44 when the implant has been combined with, that is, assembled with, the inserter 40, while still providing sufficient columnar strength to transfer enough force to drive the implant into bone. Thus, when tip portion 48 has been placed within an implant lumen, preferably substantially along its entire length, and more preferably fully placed such that shoulder 46 abuts a feature on the implant such as proximal end 62 of suture anchor 60, FIG. 4, the combination of tip portion 48 and the implant provide mutual support and reinforcement during insertion into bone.

Preferably, compliant region 44 provides a flexible bending zone and reduces the rigidity of the inserter against side loading during hammering of shaft 42 to drive anchor 60 into bone. Typical glenoid bone has a hardness of 55-D. It is desirable to have the compliant region 44 yield before the anchor or the bone become damaged. In other constructions, compliant region 44 is formed of a more malleable material, with dimensions adjusted to deliver the performance characteristics described above.

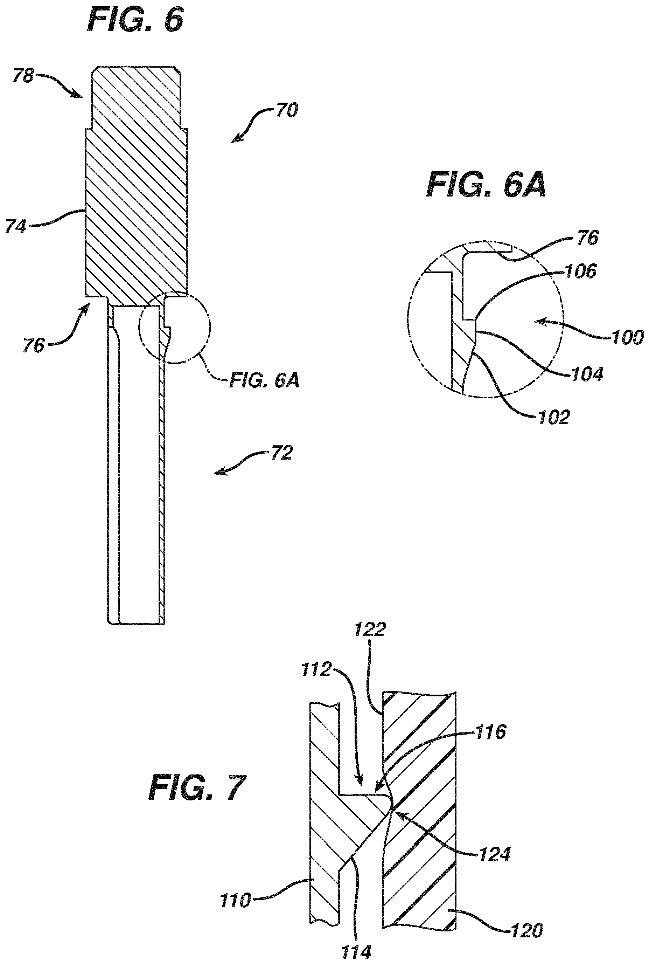

A distal portion of another exemplary inserter 70 is shown in FIGS. 5 and 6 having a tip portion 72 attached to a cylinder 74 with a shoulder 76. Cylinder 74 and a reduced-diameter portion 78 are shown as solid but may be cannulated in other constructions. Also, portion 78 may define an internal socket or external threads to mate with an elongated drive shaft. Portion 78 serves as a compliant region in one construction and, in another construction, a separate, more bendable element is connected to serve as the compliant region.

Tip portion 72 defines an opening 82 and faces 84, 86, 88, 90 and 92 for engaging corresponding features within the lumen of an implant. In other constructions, opening 82 is a solid face so that tip 72 defines a hexagonal shape or other polygon in cross-section.

Tip portion 72 further defines an implant retention element 100 which is shown enlarged in FIG. 6A as a projection having a smooth leading distal edge 102, a facet 104, and a sharply-defined transition 106 at a trailing edge. Similarly, FIG. 7 is a schematic cross-sectional view of a barb-like anchor retention element 112 of a tip portion 110 interacting with an internal wall 122 of an implant 120. Features 100 and 112 are examples of one or more possible additions to the distal tip portions of the inserters of FIGS. 1 and 3.

As shown in FIG. 7, barb 112 is of sufficient height to provide an interference fit against the inside wall 122 of the implant 120. Preferably, tip portion 110 and barb 112 are constructed with high stiffness in comparison to the implant 120. In response to the interference fit of the two objects, the implant 120 deforms, preferably elastically, as shown by deformation 124 in the vicinity of barb 112. A smooth lead-in, such as for leading edge 102, FIG. 6A, and leading edge 114, FIG. 7, permits releasable joining of tip portion 110 and implant 120 without skiving or plastic deformation of implant 120. Frictional reaction loading between tip portion 110 and implant 120 is increased by element 112 to minimize unintended separation. Further, the sharp transition edge 106, FIG. 6A, and edge 116, FIG. 7, "bites" into wall 122 at deformation 124 to further resist separation.

After implant 120 is fully installed into bone or other hard substrate, a user of the inserter must provide sufficient proximally-directed withdrawal force on tip portion 110 to overcome frictional resistance and any resistance to shear imparted from implant 120 onto element 112. Preferably, the withdrawal force is less than the force required to separate implant 120 from its substrate or to reduce the fixation between the implant 120 and the substrate. The geometric dimensions and tolerances of elements 100 and 112 may be tailored as desired to optimize these factors.

## Claims

1. An inserter (10) for an implant (15) comprising:
an elongated, substantially rigid shaft (12) having a proximal surface which is capable of receiving a driving force;
a substantially rigid tip portion (14) having a distal end and a proximal end, and at least the distal end being capable of being placed within an implant (15); and
a compliant region (16) disposed between the tip portion and the shaft which requires less lateral force to bend than the tip portion; **characterised in that**:
the compliant region includes a bellows-type structure (28).

2. The inserter of claim 1 wherein the tip portion is capable of providing internal support for the implant during insertion into bone.

3. The inserter of claim 1 or claim 2 wherein the compliant region is formed of a material that is more malleable than that of the tip portion.

4. The inserter of any preceding claim wherein the distal tip is capable of applying torque to the implant.

5. The inserter of claim any preceding claim further including an implant retention element (100) carried by the tip portion.

6. The inserter of claim 5 wherein the retention element includes a laterally-extending projection formed on the tip portion.

7. The inserter of claim 6 wherein the projection has a smooth distal leading edge (102) and a sharply-defined transition (106) at a trailing edge.

8. The inserter of any preceding claim wherein the inserter is sterile and further including a sterility maintaining package which surrounds the inserter to maintain sterility of the inserter until the package is opened.

9. A combination comprising:
a suture anchor (15) having a distal end and a proximal end, and defining a lumen open at the proximal end;
the inserter (10) of any preceding claim;
wherein at least the distal end of the substantially rigid tip portion is capable of being placed within the lumen of the suture anchor to provide internal support for the suture anchor during insertion into bone;
wherein the inserter includes a distal-facing shoulder (76) at the proximal end of the tip portion capable of engaging the proximal end of the suture anchor; and
the compliant region is disposed between the shoulder and the shaft and requires less lateral force to bend than the tip portion, at least when the tip portion has been placed within the suture anchor.

10. The combination of claim 9 further including an anchor retention element (100) formed on the tip portion.

11. The combination of claim 10 wherein the suture anchor is formed of a material more elastically deformable than the anchor retention element.

12. The combination of claim 9 further including a sterilizable package which surrounds at least the inserter to maintain sterility of at least the inserter until the package is opened.

## Patentansprüche

1. Einführer (10) für ein Implantat (15), umfassend:
einen länglichen, im Wesentlichen starren Schaft (12), der eine proximale Fläche hat, die eine Treibkraft aufnehmen kann,
einen im Wesentlichen starren Spitzenabschnitt (14), der ein distales Ende und ein proximales Ende hat, und wobei mindestens das distale Ende in einem Implantat (15) platziert werden kann, und
einen nachgiebigen Bereich (16), der zwischen dem Spitzenabschnitt und dem Schaft angeordnet ist und bei dem zum Biegen weniger seitliche Kraft aufgewendet werden muss als beim Spitzenabschnitt, **dadurch gekennzeichnet, dass**:
der nachgiebige Bereich eine balgartige Struktur (28) umfasst.

2. Einführer nach Anspruch 1, wobei der Spitzenabschnitt interne Stützung für das Implantat während des Einführens in Knochen bereitstellen kann.

3. Einführer nach Anspruch 1 oder Anspruch 2, wobei der nachgiebige Bereich aus einem Material gebildet ist, das formbarer ist als das des Spitzenabschnitts.

4. Einführer nach einem vorhergehenden Anspruch, wobei die distale Spitze das Implantat mit Drehmoment beaufschlagen kann.

5. Einführer nach einem vorhergehenden Anspruch, der ferner ein vom Spitzenabschnitt getragenes Implantatrückhalteelement (100) aufweist.

6. Einführer nach Anspruch 5, wobei das Rückhalteelement einen am Spitzenabschnitt ausgebildeten, sich lateral erstreckenden Vorsprung aufweist.

7. Einführer nach Anspruch 6, wobei der Vorsprung einen glatten distalen vorderen Rand (102) und einen scharf definierten Übergang (106) an einem hinteren Rand hat.

8. Einführer nach einem vorhergehenden Anspruch, wobei der Einführer steril ist und ferner eine Sterilität aufrechterhaltende Packung aufweist, die den Einführer umgibt, um die Sterilität des Einführers bis zum Öffnen der Packung aufrechtzuerhalten.

9. Kombination, umfassend:
einen Fadenanker (15), der ein distales Ende und ein proximales Ende hat und ein am proximalen Ende offenes Lumen definiert;
den Einführer (10) nach einem vorhergehenden Anspruch,
wobei mindestens das distale Ende des im Wesentlichen starren Spitzenabschnitts in das Lumen des Fadenankers platziert werden kann, um interne Stützung für den Fadenanker während des Einführens in Knochen bereitzustellen,
wobei der Einführer am proximalen Ende des Spitzenabschnitts eine nach distal gewandte Schulter (76) aufweist, die das proximale Ende des Fadenankers in Eingriff nehmen kann, und
der nachgiebige Bereich zwischen der Schulter und dem Schaft angeordnet ist und bei dem zum Biegen weniger seitliche Kraft aufgewendet werden muss als beim Spitzenabschnitt, zumindest, wenn der Spitzenabschnitt in den Fadenanker platziert worden ist.

10. Kombination nach Anspruch 9, die ferner ein am Spitzenabschnitt gebildetes Ankerrückhalteelement (100) aufweist.

11. Kombination nach Anspruch 10, wobei der Fadenanker aus einem Material gebildet ist, das elastisch verformbarer als das Ankerrückhalteelement ist.

12. Kombination nach Anspruch 9, die ferner eine sterilisierbare Packung aufweist, die mindestens den Einführer umgibt, um die Sterilität mindestens des Einführers bis zum Öffnen der Packung aufrechtzuerhalten.

## Revendications

1. Dispositif d'insertion (10) pour un implant (15) comprenant :
une tige allongée substantiellement rigide (12) ayant une surface proximale apte à recevoir une force d'entraînement ;
une portion de pointe substantiellement rigide (14) ayant une extrémité distale et une extrémité proximale, et au moins l'extrémité distale étant apte à être placée à l'intérieur d'un implant (15) ; et
une région souple (16) disposée entre la portion de pointe et la tige, qui a besoin de moins de force latérale pour fléchir que la portion de pointe ; **caractérisé en ce que** :
la région souple comprend une structure de type soufflet (28).

2. Dispositif d'insertion selon la revendication 1, la portion de pointe étant apte à fournir un support interne pour l'implant au cours de l'insertion dans l'os.

3. Dispositif d'insertion selon la revendication 1 ou 2, la région souple étant formée d'un matériau qui est plus malléable que celui de la portion de pointe.

4. Dispositif d'insertion selon l'une quelconque des revendications précédentes, l'extrémité distale étant apte à appliquer un couple à l'implant.

5. Dispositif d'insertion selon l'une quelconque des revendications précédentes, comportant en outre un élément de retenue d'implant (100) porté par la portion de pointe.

6. Dispositif d'insertion selon la revendication 5, l'élément de retenue comportant une saillie s'étendant latéralement formée sur la portion de pointe.

7. Dispositif d'insertion selon la revendication 6, la saillie présentant un bord d'attaque distal lisse (102) et une transition définie de manière vive (106) au niveau d'un bord de fuite.

8. Dispositif d'insertion selon l'une quelconque des revendications précédentes, le dispositif d'insertion étant stérile et comportant en outre un emballage conservant la stérilité qui entoure le dispositif d'insertion pour maintenir la stérilité du dispositif d'insertion jusqu'à l'ouverture de l'emballage.

9. Combinaison comprenant :
un ancrage pour suture (15) ayant une extrémité distale et une extrémité proximale, et définissant une lumière ouverte à l'extrémité proximale ;
le dispositif d'insertion (10) selon l'une quelconque des revendications précédentes ;
au moins l'extrémité distale de la portion de pointe sensiblement rigide étant apte à être placée à l'intérieur de la lumière de l'ancrage pour suture pour fournir un support interne à l'ancrage pour suture au cours de l'insertion dans l'os ;
le dispositif d'insertion comportant un épaulement orienté distalement (76) à l'extrémité proximale de la portion de pointe, apte à s'engager avec l'extrémité proximale de l'ancrage pour suture ; et
la région souple étant disposée entre l'épaulement et la tige et ayant besoin de moins de force latérale pour fléchir que la portion de pointe, au moins lorsque la portion de pointe a été placée à l'intérieur de l'ancrage pour suture.

10. Combinaison selon la revendication 9, comportant en outre un élément de retenue d'ancrage (100) formé sur la portion de pointe.

11. Combinaison selon la revendication 10, l'ancrage pour suture étant formé en un matériau plus déformable élastiquement que l'élément de retenue d'ancrage.

12. Combinaison selon la revendication 9, comportant en outre un emballage pouvant être stérilisé, qui entoure au moins le dispositif d'insertion pour maintenir la stérilité au moins du dispositif d'insertion jusqu'à l'ouverture de l'emballage.
